# EUROPEAN PATENT SPECIFICATION

(11) **EP 2 233 108 B1**
(45) Date of publication and mention of the grant of the patent: **29.04.2015**
(21) Application number: 09155873.4
(22) Date of filing: 23.03.2009
(51) Int. Cl.: A61C 8/00, A61F 2/30, A61B 17/86

(54) **Bone fixture**
Knochenaufsatz
Implant osseux

(43) Date of publication of application: 29.09.2010
(73) Proprietor: Dentsply IH AB, 431 21 Mölndal (SE)
(72) Inventor: Holmström, Johan, SE-428 37, Kållered (SE)
(74) Representative: Somlo, Tommy

(56) References cited:
- EP-A- 0 714 643
- EP-A- 1 440 669
- WO-A-03/055405
- WO-A-2004/058091
- WO-A-2004/098442
- DE-A1-102006 011 629
- DE-U1- 29 703 296
- FR-A- 2 610 512
- US-A1- 2004 170 946
- US-A1- 2006 204 930
- US-B1- 6 419 491

## Description

### Technical field

The present invention relates to a fixture for insertion into a human bone.

### Background

A frequent way today to restore a damaged limb, such as lost tooth, damaged hip joint or finger is to install a fixture in the adjacent bone tissue and replace the damaged parts. In this respect, for a successful result, the fixture should become fully stable and correctly joined to the bone. The term osseointegration is used for this joining effect, the basic meaning of this term being the bone tissue growth into the fixture surface. The two major contributors to this joint are a mechanical joint and an organic joint. The former being generally influenced by the macro geometry of the bore into which the fixture is installed, and by the macro geometry of the fixture, and is a direct effect of how well these two work together. The latter one being a continuously evolving and developing effect, particularly the time immediately after installation, and being generally influenced by how well the micro surface structure of the fixture interacts with the bone tissue.

Due to ingrowth there will be an interlocking effect between the bone and the fixture. Also, the mechanical joint is developed over time since the bone tissue, under ideal conditions, may grow into surface cavities of the fixture, and grow into voids left between the fixture and the bore after installation.

Interaction of mechanical and organic aspects will affect the bone ingrowth and joint between the bone and the projecting surface structure of the fixture. The projecting surface structure may be in the form of threads, annular ridges, lines or patterns of beads etc. Further a blasted surface of the fixture will provide advantageous conditions for this process.

From a patient perspective it is desirable to have a prosthesis in place as soon as possible. For instance, for a tooth replacement, mastication with the prosthesis transmits an axial load onto the fixture. A common understanding is that the installed fixture should not be subjected to fully functional load before sufficient ingrowth of bone tissue has occurred. There are situations in which a limited load may be allowed during the healing period, and there are other situations in which any load should be avoided during the healing period. This understanding is not only applicable to dental fixtures, but also to other fixtures. It would be desirable to shorten the time for ingrowth of bone tissue in order to allow a patient to obtain a usable prosthesis as soon as possible.

US 6,419,491 B1 discloses a dental implant system with repeating microgeometric surface patterns. According to US 6,419,491 B1, the microgeometric surface patterns are intended to promote the bone growth and discourage the growth of soft tissue to achieve secure fixation of the implant surface to bone tissue.

WO 2004/098442 A1 discloses a bone implant having a screw thread. Groove-like recesses are made in the screw thread for promoting attachment of osteons to the implant surface.

### Summary of the Invention

The present invention is based on an insight that the time for ingrowth of bone tissue may be positively affected by allowing for a blood distribution in a direction extending from the core of the fixture towards the surrounding bone (or in a direction extending from the surrounding bone towards the core of the fixture). In particular, it has been realized that by providing an at least partly radially extending gap on a projecting surface structure of the fixture, the amount of initial bone resorption may be reduced while providing a larger area stimulating ingrowth of bone tissue. Furthermore, it has been realized that, in case the projecting surface structure is provided in the form of threads, channels along thread flanks of an installed fixture may be used for transporting blood and thus stimulating the ingrowth of bone tissue. The invention is also based on the understanding that a small load on the fixture may give rise to a pumping effect, which normally takes place in bone, and which may be increased when the fixture is subjected to load during the healing period.

According to one aspect of the invention, a fixture for insertion into a human bone is provided, as defined in claim 1.

Thus, the distance between the channel bottom and the core varies with the extension of the channel along the surface structure. Since the channel will extend over the surface of the surface structure at varying distance from the core of the fixture, it may be designed so that it stimulates bone ingrowth over a large area of the surface of the surface structure. Additionally, the presence of said one or more channels increases the total surface area of the fixture compared to a fixture without channels. The increased surface area enables an increased interlocking effect between the bone and the fixture. Furthermore, the presence of said one or more channels may facilitate/speed up the healing process in low-quality bone tissue.

The path may be substantially straight or may have a curved extension, it may even twist and turn as it winds its way from the core towards the structure top. The channel may extend along part or parts of the path or along the entire path. According to the invention, the channel continues over and past the structure top and then extends down towards the core again (either towards a different structure bottom location or back towards the same structure bottom location). The radial distance between a longitudinal axis of the fixture and the channel bottom increases as the channel passes along said path towards the structure top. Conversely, the radial distance between the longitudinal axis of the fixture and the channel bottom decreases as the channel passes along said path towards the structure bottom or the core of the fixture.

According to at least one example embodiment, said at least one channel comprises two or more channels extending in parallel with each other. This allows a large area of the projecting surface structure to be provided with blood-distributing channels. Said two or more channels may suitably be arranged in the form of spirals around the core or a geometrical longitudinal axis of the fixture. Alternatively, they may describe an annular path around the fixture.

According to at least one example embodiment, said at least one channel comprises two or more channels extending non-parallelly relative to each other. This may also allow a large area of the projecting surface structure to be provided with blood-distributing channels. The extensions of the channels may be completely separated from each other. However, they may, as an alternative be converging towards each other. For instance, the channel may emanate from a common point or may even cross each other's paths. In this way, a system of interconnected blood-distributing channels may be provided on the surface of the surface structure. A fixture may be provided with one or more of the exemplified extension alternatives. Furthermore, two or more non-parallelly extending channels may be combined with parallelly extending channels.

According to at least one example embodiment, said at least one channel is a plurality of channels that form a square-like or diamond-like pattern. In addition to allowing a large area of the surface of the projecting surface structure to be covered, by having the channels interconnected with each other in a grid-like manner, the blood distribution may be further promoted.

Although, the channel or channels may advantageously describe a path directed along the length of the fixture, according to at least one example embodiment, said at least one channel extends substantially in the circumferential direction of the fixture. According to at least one example embodiment the at least one channel forms an annular path around the longitudinal axis and the core of the fixture. In other words, the channel may be regarded as a circle-shape which is projected onto the surface of the core and onto the surface of said surface structure. This is further partly reflected in at least one example embodiment, according to which the channel represents a linear, a circular or a helical shape which has been projected onto the surface of the fixture, i.e. onto the surface of the core and the surface of the projecting surface structure.

Said at least one channel may be in the form of one or more short discrete channel portions, provided at one or more locations on the fixture. The channel portions may be scattered or may form one or more broken lines. Alternatively, said at least one channel may be in the form of one or more long continuous channels extending over a plurality of surface structure tops and bottoms, thus describing a roller-coaster path along the fixture surface. This latter alternative is reflected in at least one example embodiment, according to which the radial distance between the core of the fixture (or longitudinal axis of the fixture) and the channel bottom alternatingly increases and decreases along the extension of the channel.

According to the invention, the depth of the at least one channel is 0.05- 0.15 mm. According to at least one example embodiment, the depth of the at least one channel is 0.05 mm - 0.1 mm.

According to at least one example embodiment, the depth of said at least one channel is smaller than the height of the surface structure, suitably the depth of said at least one channel is 50% or less than 50% of the height of the surface structure, for instance 30% or less than 30% of the height of the surface structure, such as 15%-30% of the height of the surface structure. The depth of said at least one channel may even be greater than 50% of the height of the surface structure, e.g. in case of a surface structure projecting no more than 0.2 mm from the core of the fixture, e.g. a surface structure in the form of small threads or circumferentially-oriented ridges or raised strips.

According to at least one example embodiment, the fixture comprises an anchoring portion adapted to be fully inserted into the bone, wherein more than 1 % of the total circumferential surface area of the anchoring portion is provided with/constituted by said at least one channel, suitably more than 5%, for instance more than 10%, such as more than 20%. The anchoring portion may constitute the entire fixture. Thus, the fixture would be completely inserted into the bone, while a superstructure may be connectible to the fixture to project from the bone. Alternatively, the fixture may in addition to the anchoring portion comprise a projecting portion, which is adapted to project from the bone while the anchoring portion is located in the bone.

According to the invention, said circumferentially-oriented roughness is in the form of one or more helical threads.

According to the invention, the fixture comprises an external thread having a thread profile, wherein said at least one channel is provided in the surface of the thread, wherein the channel bottom substantially follows the variations of the thread profile relative to said core of the fixture. The fixture may be considered to have a geometrical longitudinal axis, wherein the external thread, in the axial direction, has alternating thread bottoms and thread tops, wherein at least some of the thread bottoms are located in the surface of the core of the fixture, wherein the distance between the channel bottom and said core varies with the extension of the channel along the thread. In other words, when the radial distance from the longitudinal axis to the thread profile increases, the radial distance from said axis to the channel bottom also increases. Conversely, when the radial distance from the longitudinal axis to the thread profile decreases, the radial distance to the channel bottom also decreases.

According to at least one example embodiment, the thread extends in the form of a spiral around the longitudinal axis of the fixture, wherein the extension of the channel is nonparallel with said spiral. Thus, the channel will describe a path which extends in a direction between thread top(s) and thread bottom(s). The channel itself may be in the form of a spiral around the longitudinal axis, however, its lead will then be different from the lead of the thread spiral. Suitably, a plurality of spiral-shaped channels may be provided. Some of the channels may cross each others' paths.

Although the general inventive idea is applicable in various types of fixtures for stimulating blood distribution in various types of bone tissues, for simplicity, the following discussion will mainly be related to dental fixtures.

Thus, according to at least one example embodiment, the fixture is a dental fixture for insertion into a human jawbone. Suitably, the fixture may be a screw-type fixture which is inserted by means of rotation into the bone tissue.

A dental fixture is for use as the anchoring member of a dental prosthesis. To this end, the dental fixture according to at least one example embodiment, is insertable (such as by means of rotation) into a bore-hole drilled into the bone tissue of a jawbone (maxilla or mandible) at a site where the dental prosthesis is required. For screw-type fixture embodiments, the bore-hole may be provided with internal threads in advance or may be left untapped with the fixture provided with a self-tapping capacity, e.g. by the provision of one or more axially-extending cutting recesses, edges or notches, etc in the fixture thread. For instance, an apical end portion of the fixture may be provided with 2-4 cutting recesses, such as 3 cutting recesses. Other number of cutting recesses is readily conceivable.

A superstructure for connecting a prosthetic part to the fixture may comprise an abutment, spacer or other transmucosal component which engages to the dental fixture to bridge the gingiva overlying the maxilla or mandible. The prosthetic part, e.g. a crown, bridge or denture may be secured to the abutment. There are various other forms that the superstructure can take. For instance, the prosthetic part may be secured directly to the dental fixture. A dental implant may thus comprise an abutment connected to the dental fixture, or just the dental fixture without an abutment.

The term "coronal" is here and throughout this application used to indicate a direction towards a head end or trailing end of the dental implant. For instance, in a situation where an abutment is connected to the dental fixture, the coronal direction of the abutment would be a direction towards the part of the abutment being directed away from the fixture. Conversely, the term "apical" indicates a direction towards an insertion end of the component. Thus, apical and coronal are opposite directions. Furthermore, the term "axial direction" or "axially" is used throughout this application to indicate a direction taken from the coronal end to the apical end, or vice versa.

A blind bore or socket may extend apically into the fixture body from the coronal end to an end surface in-between the apical and coronal ends of the fixture body for a superstructure to be secured to the fixture. The socket may comprise an internally-threaded section for screw connection of the superstructure to the fixture. A rotational lock for the superstructure may be provided in the socket, such as an internal polygonal side wall, e.g. hexagonal, or alternatively one or more protrusions from or indentation in the wall of the socket. A section of the socket, such as the coronal section, may be tapered towards the apical end. The tapered section is suitably arranged coronally of the internally-threaded section.

The dental fixture may be used in a one stage procedure or a two stage procedure. In a one stage procedure a healing or temporary abutment is connected to the fixture to form the gingival tissue, and after a healing period the healing or temporary abutment is replaced by a permanent abutment. For a two stage procedure the fixture is provided with a cover screw and the gingival tissue is sutured over the fixture and cover screw, and after a healing period the tissue is opened up and an abutment is connected to the fixture after removal of the cover screw.

A conceivable alternative to having an abutment connected to the dental fixture is to have a one-piece implant, wherein a portion of the implant is embedded in bone tissue, while another portion of the implant extends from the bone tissue across the gingiva.

The dental fixture may have a conically tapering end portion which tapers towards the coronal end. The axial extent of this coronal end portion is small compared to the total length of the fixture, as an example no more than 4 % of the total length, such as in the range of 1.5%-3.7%. The coronal end portion may suitably be provided without a threaded surface, e.g. having a smooth or a blasted surface.

The dental fixture may have a substantially flat coronal end surface which is perpendicular to the longitudinal axis of the fixture. Alternatively, the coronal end surface may have a sloped contour relative to the longitudinal axis of the fixture, e.g. such that when positioned within the jawbone the length of the fixture is larger on a lingual side and shorter on a buccal side of the fixture. Another alternative is a saddle-shaped coronal end surface.

The length of the dental fixture may, for instance, be in the range of 5-19 mm, depending on the clinical situation. The outer diameter of the fixture may suitably be in the range of 2-6 mm, such as 3-5 mm.

The dental fixture may be substantially cylindrical or slightly tapering from the coronal end towards the apical end. If the fixture has a slight conicity, the core of the fixture and the outer periphery defined by e.g. thread tops may have the same or different angle of taper. Furthermore, the core of the fixture may be cylindrical while the thread tops describe a conicity or, conversely, the core of the fixture may be tapered while the thread tops describe a generally cylindrical geometry. Alternatively, the fixture may comprise a combination of one or more cylindrical and/or one or more tapering portions. Thus, one or more portions of the fixture may have e.g. thread tops lying in a common imaginary cylindrical surface, which cylindrical surface is parallel with the longitudinal axis of the fixture. Alternatively or additionally, one or more portions of the fixture may have thread tops lying in an imaginary conical surface which in the apical direction is tapering towards the longitudinal axis.

The fixture may comprise one or more external thread spirals, i.e. a multi-start thread.

The term "pitch" is used to indicate the axial distance between adjacent tops of a threading. The term "lead" is used to indicate the distance advanced parallel to the longitudinal axis when the fixture is turned one revolution, i.e. it corresponds to the pitch multiplied with the number of thread spirals. For a single thread spiral having a constant pitch, the lead is equal to the pitch; for a double thread spiral, the lead is twice the pitch.

The term "microthread" is used to indicate a thread having a height which is no greater than 0.2 mm. According to at least one example embodiment, the fixture is provided with microthreads having a height in the range of 0.02-0.2 mm, such as 0.05-.015 mm, for instance 0.1 mm. The term "macrothread" is used to indicate a thread having a height which is greater than 0.2 mm. According to at least one example embodiment, the fixture is provided with macrothreads having a height in the range of 0.25-0.35 mm, such as 0.3 mm.

Suitably, microthreads may be located coronally of macrothreads. For instance, microthreads may be arranged to engage dense cortical bone and macrothreads may be arranged to engage porous spongious/cancellous bone. The lead of a microthread suitably corresponds to the lead of a macrothread. The macrothread pitch may, as an example, be 2-4 times, such as 3 times, the pitch of the microthreads. The pitch (top-to-top spacing) at a fixture portion provided with microthreads may be around 0.20-0.24 mm. The pitch (top-to-top spacing) at a fixture portion provided with macrothreads may be around 0.60-0.72 mm. As an alternative (or in addition) to microthreads, the fixture may be provided with annular ridges coronally of the macrothreads.

Microthreads can be regarded as defined, oriented roughness. A non-oriented roughness having smaller dimensions, for instance obtained by blasting, etching, etc. may be superimposed on microthreads as well as on macrothreads.

Said at least one channel may, optionally, be provided with a modified surface, such as by blasting, etching, or any other suitable type of surface modification.

A thread profile comprises two flanks, a top radius R, at the apex formed between the intersection of said two flanks, a bottom radius r formed between two adjacent threads, said flanks forming an angle v with a plane which is perpendicular to a cross section of said thread and perpendicular to a plane which is a tangent to the surface of the fixture body, said profile further having a height D. Suitably for 10° ≤ v < 35 °, R is greater than 0.4 x D and, for 35° ≤ v < 55 °, R is greater than 0.2 x D.

### Brief description of the drawings

Fig. 1 illustrates a fixture according to at least a first example embodiment of the present invention.
Fig. 2 illustrates a fixture according to at least a second example embodiment of the present invention.
Fig. 3 illustrates a fixture according to an example embodiment which does not fall within the scope of the claims.
Fig. 4 illustrates a portion of a fixture according to an example embodiment which does not fall within the scope of the claims.
Figs. 5a and 5b illustrate a fixture according to at least a third example embodiment of the invention.
Fig. 6 illustrates a fixture according to at least a fourth example embodiment of the invention.
Fig. 7 illustrates a fixture according to at least a fifth example embodiment of the invention.
Fig. 8 illustrates a fixture according to an example embodiment which does not fall within the scope of the claims.
Fig. 9 illustrates a fixture according to an example embodiment which does not fall within the scope of the claims.

### Detailed description of the drawings

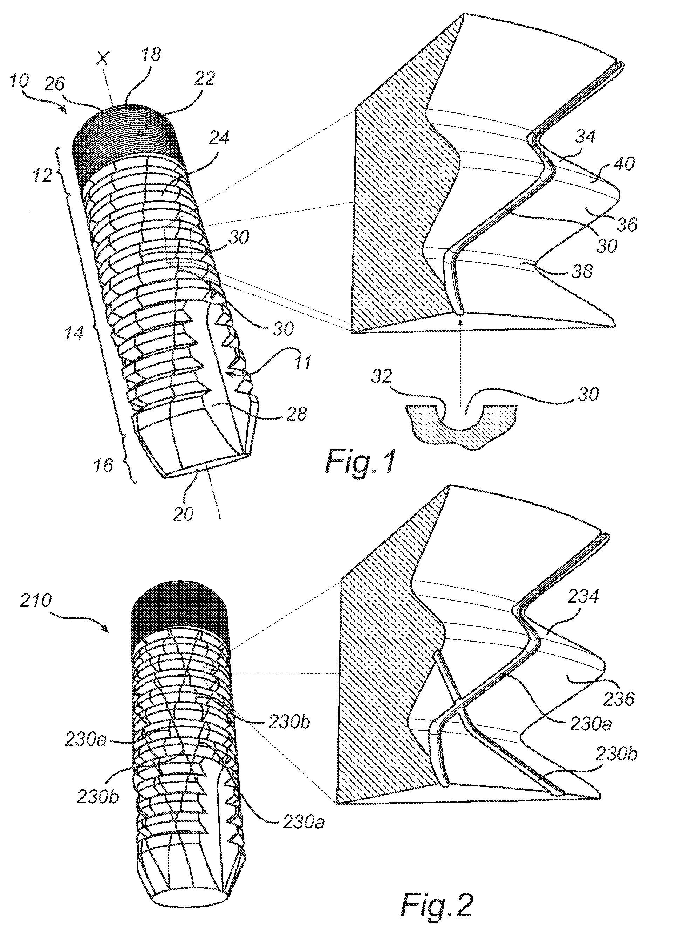

Fig. 1 illustrates a fixture 10 according to at least a first example embodiment of the present invention. The fixture 10 has a coronal portion 12 extending apically from a coronal end 18 of the fixture 10, and an apical portion 16 extending coronally from an apical end 20 of the fixture 10. An intermediate portion 14 extends between the coronal portion 12 and the apical portion 16.

The apical portion 16 has a conicity tapering towards the apical end 20 of the fixture 10 to ease insertion of the fixture 10 into a bore-hole. The angle of taper relative to the longitudinal axis X of the fixture 10 may, for instance, be about 10°-20°, such as 15°. Although the figure illustrates a non-threaded apical portion 16, in an alternative embodiment the apical portion of the fixture may be provided with an external thread. Whether provided with thread or not, the apical portion 16 may optionally, similarly to the coronal and/or the intermediate portion, further be provided with a blasted surface structure.

The fixture 10 has a core 11 from which a surface structure projects, in the illustrated example being in the form of threads 22, 24.

The coronal portion 12 is herein illustrated as being at least partly provided with microthreads 22, having three thread spirals, although another number is conceivable, such as 1, 2, 4 or more. Although microthreads have been illustrated, according to at least an alternative example embodiment the coronal portion is at least partly provided with macrothreads 24, similarly to the intermediate portion 14, either as a separate thread spiral or as a continuation of the thread spiral at the intermediate portion 14. According to at least another alternative example embodiment, instead of microthreads, the coronal portion may be provided with a plurality of annular ridges, which to the naked eye could give the same visual appearance as microthreads. Other conceivable alternatives are circumferential lines of beads or non-oriented/randomly provided projections such as bulges.

In the illustrated example embodiment, the macrothreads 24 at the intermediate portion 14 has the same lead as the microthreads 22 at the coronal portion 12. However, the pitch of the macrothreads 24 is three times the pitch of the microthreads 22, since the microthreads 22 comprise three thread spirals.

The length of the herein illustrated coronal portion 12 may be about 1-2 mm, such as 1.5 mm. However, shorter or longer lengths are readily conceivable. The relative length of coronal portion 12 may also be selected from a wide range, such as 5-50% of the total length of the fixture 10, e.g. 10-20%.

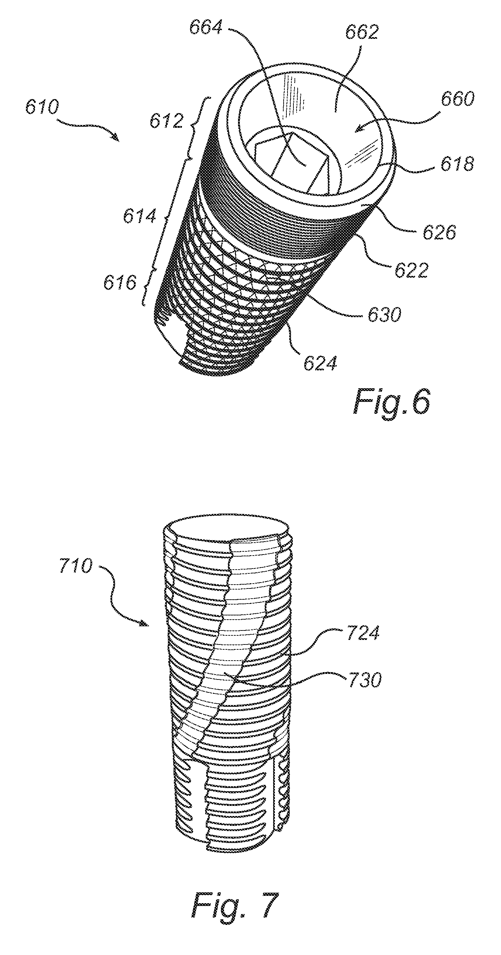

The coronal portion 12 comprises a tapering end portion 26, which tapers towards the coronal end 18 of the fixture 10. The tapering end portion 26 is no more than 4% of the total length of the fixture 10. The surface of the tapering portion 26 may be non-threaded, either smooth or blasted. Such a tapering portion 26 is more clearly visible in the example embodiment of Fig. 6 (reference numeral 626).

Continuing with Fig. 1, the intermediate portion 14 comprises macrothreads 24. Although illustrated as having one thread spiral, the intermediate portion 14 may alternatively have two or more thread spirals. Similarly, although illustrated as having a substantially straight cylindrical shape, the intermediate portion 14 may have a slight conicity, i.e. a slightly tapering shape towards the apical portion 16, in which case the angle of taper may e.g. be 3° or less, such as about 1°-2°.

A cutting recess 28 or groove extends from the apical end 20 into the intermediate portion 14. Although only one cutting recess 28 is illustrated in the figure, the fixture 10 may have more, such as two, three or four cutting recesses 28, suitably symmetrically positioned about the circumference of the apical end 20 of the fixture 10 for self-tapping of the fixture 10 when being screwed/rotated into the bore-hole provided in the maxilla or mandible.

The apical portion 16 and the intermediate portion 14 are provided with a plurality of shallow channels 30 at the external fixture surface. The channels 30 are herein illustrated as extending in parallel to each other. The channels 30 are herein also illustrated as having a main direction of extension somewhat inclined and non-parallel relative to the longitudinal axis X of the fixture 10. However, other inclinations are readily conceivable, and they may or may not be parallel with each other or the longitudinal axis X of the fixture 10. The channels 30 may even be inclined to such extent that they form spirals around the longitudinal axis X of the fixture 10.

As illustrated in the enlarged cut-out portion of Fig. 1, the channels 30 form roller-coaster like paths as they go from a coronal flank 34 to an apical flank 36 and continues to the next coronal flank and next apical flank, etc. In other words, the channels 30 and channel bottoms 32 form paths which extend alternatingly and repeatedly along thread bottoms 38 and thread tops 40. Thus, in accordance with at least one aspect of the invention, the channel bottom 32 extends along a path extending from the core 11 of the fixture 10 towards a structure top (in this example: a thread top 40), wherein the depth of the channel 30 is smaller than the height of the surface structure (in this example: the height of the thread 24).

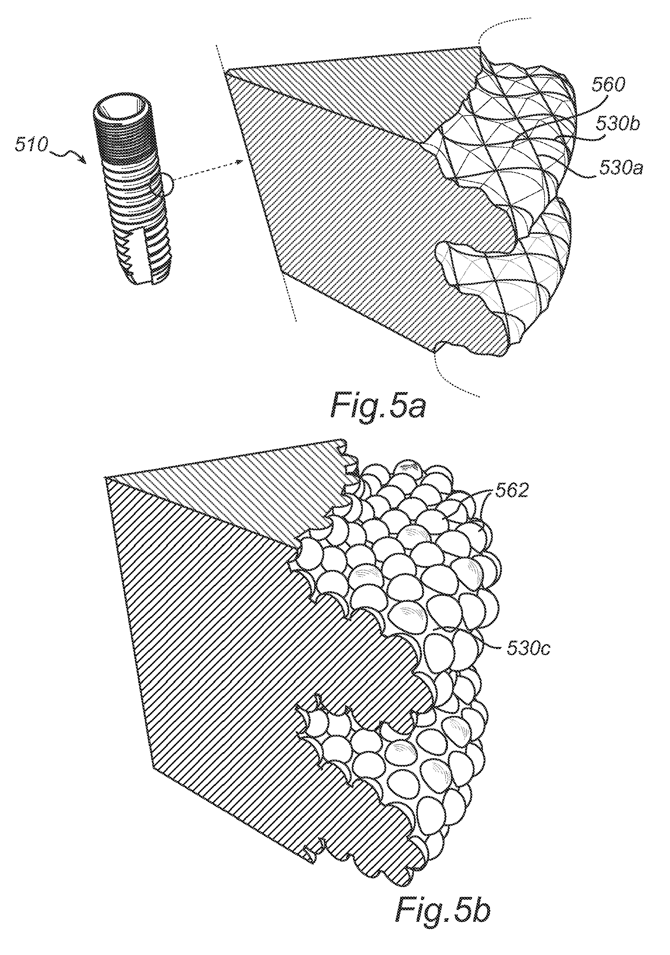

When the fixture 10 has been installed and the threads have cut into and engaged the bone, the part of the bone tissue which is in contact with the thread is likely to initially be resorbed. However, due to the presence of the channels 30 there will also directly after installation, be small gaps between the thread and the bone tissue. In those gaps between the channel bottom 32 and the surrounding bone blood is allowed to flow and stimulate in-growth of bone tissue. In some cases, immediately after installation, the presence of blood is highest near the core of the fixture body, i.e. at the thread bottoms 38, in which case the channels 30 extending along the flanks 34, 36 radially towards the periphery of the threads will advantageously distribute the blood in that direction. The presence of blood channels and the process of early in-growth is also believed to stimulate the other parts of the bone tissue, thereby reducing the bone resorption and the time for satisfactory healing-in of bone tissue. Thus, simultaneously with potential resorption of some bone tissue, other bone tissue will grow into the gaps and the channels 30. Eventually the bone tissue at the potentially resorbed areas will regrow to fully stabilize the fixture 10 in the jawbone. By having the channels 30 extending along the flanks 34, 36, following the thread profile, a large area for stimulating in-growth may be accomplished. Although Fig. 1, for exemplifying purposes, has been illustrated with sparsely provided channels 30 on the fixture surface, it would be conceivable to have the channels 30 located much closer to each other and crossing each others' paths, thereby further increasing the bone in-growth stimulating area, e.g. as illustrated in the enlarged cut-out part of Figs. 5a and/or 5b.

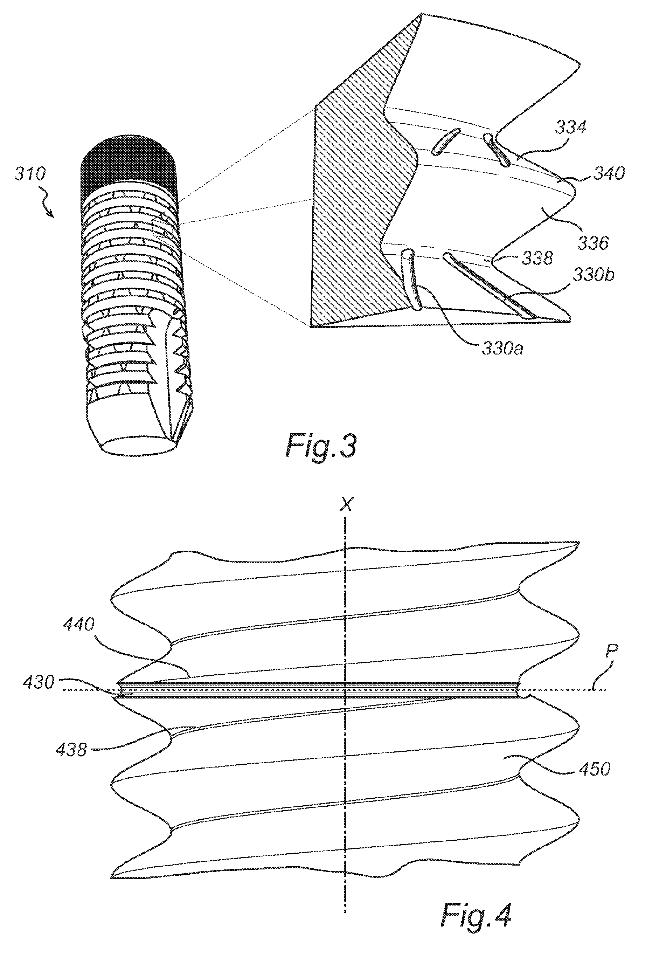

Although Fig. 1 illustrates the provision of channels 30 at the apical portion 16 and although the apical portion 16 may be provided with threads, an alternative would be to only have channels 30 at the intermediate portion 14 (see e.g. example embodiment of Fig. 3). Furthermore, it would be conceivable to have only part of the length of the intermediate portion 14 provided with channels, such as the part of the intermediate portion extending coronally of the cutting recess 28.

Fig. 1 also illustrates a cross-sectional view of a channel 30. Although illustrated as having a U-shaped cross-section, other shapes such as V-shape, W-shape, square-shape, irregular shape, etc. are conceivable alternatives. The channel bottom 32 substantially follows the variations of the thread profile relative to the longitudinal axis of the fixture, and thus the depth of the channel 30 is smaller than the depth (height) of the thread. The depth of the channel 30 is 0.05-0.15 mm, and may more preferably be 0.05 mm - 0.1 mm. The depth of the channel 30 may suitably be 50% or less than 50% of the thread height, preferably 30% or less than 30% of thread height, more preferably 15%-30% of the thread height. As a channel 30 may have any one of a number of conceivable shapes, its depth 32, should be understood to imply the maximum depth. Furthermore, a channel may have a varying depth along its extension. Similarly, although the width of the channel has been illustrated as being constant, it is conceivable to allow its width to vary along the extension of the channel. Also, a channel may at one or more points become bifurcated or branched into several spread channels. For instance, in order to distribute blood over a large area, the "stem" channel may run from a thread bottom and then be divided into a number of "branch" channels on its way towards the thread top.

Fig. 2 illustrates a fixture 210 according to at least a second example embodiment of the present invention. In addition to a first set of channels 230a which extend substantially as illustrated in Fig. 1, the fixture 210 is also provided with a second set of channels 230b having a main direction of extension which is angled relative to the main direction of extension of the first set. The channels 230a in the first set cross the paths of the channels 230b of the second set, thereby forming a diamond-like pattern. The thread flanks 234, 236 of a fixture 210 according to Fig. 2 may thus be provided with a larger bone in-growth stimulating area compared to a fixture 10 according to Fig. 1. Furthermore, the crossing channels 230a, 230b form a system of blood canals which may further enhance the blood distribution and in-growth stimulation. The various alternatives and features discussed in relation to the fixture 10 illustrated in Fig. 1 may readily be implemented also for exemplified fixture 210 of Fig. 2.

Fig. 3 illustrates a fixture 310 according to an example embodiment which does not fall within the scope of the claims. In the illustrated embodiment, only the coronal thread flanks 334 are provided with relatively short channels 330a, 330b, while the apical thread flanks 336 are void of channels. While the channels on the coronal flanks 334 have the advantage of enabling blood distribution, the lack of channels at the apical flank 336 may have the advantage of maintaining the load bearing capability on the apical flanks 336 since the initial load is distributed over a larger area than what would be the case if the apical flanks were provided with channels.

Alternatively, only the apical thread flanks 336 may be provided with relatively short channels, while the coronal thread flanks 334 are void of channels. This may be advantageous from a bone generation promoting perspective. When the implant is subjected to an axial load, the implant is pressed in an apical direction, which means that the contact/pressure is reduced on the coronal flanks and increased on the apical flanks. Thus, the role of the coronal flanks may be reduced, and as an option, channels could be omitted on the coronal flanks. On the other hand, at the apical flank, being subjected to an increased pressure against the bone, a channel on the apical flank would result in that the bone area facing the channel is not subjected to pressure when the implant is axially loaded, and consequently reduce the risk of resorption.

Yet another alternative is to have some sub-portion provided with channels located only on the coronal flanks and another sub-portion provided with channels only located on the apical flanks. The extension of the short channels 330a, 330b on a flank 334 are herein illustrated as being nonparallel, however, parallel channels is a conceivable alternative. Although the channels 330a, 330b have been illustrated as extending all the way from thread bottom 338 to thread top 340, an alternative would be to extend only part of the distance between the thread bottom 338 and the thread top 340. The features and alternatives discussed in connection with the embodiment of Fig. 3 may be combined with above-discussed features and alternatives presented in the connection with Figs. 1 and 2.

Fig. 4 illustrates a portion of a fixture according to an example embodiment which does not fall within the scope of the claims. In the illustrated example a shallow channel 430 is located in a plane P which is perpendicular to the longitudinal axis X of the fixture, i.e. the longitudinal axis X forms a normal to the plane P. The thread spiral 450, however, having a lead, is inclined relative to said plane P, i.e. being non-parallel with the channel 430. In fact, regardless of how (at which angle) the plane P is oriented relative to the longitudinal axis, as long as the channel runs around the periphery of the implant, the thread spiral 450 will be inclined relative to the plane P. It should be noted that also in the example embodiments shown in the other figures, the thread spiral(s) is/are non-parallel with the extension of the channels. This will have the effect that the channel 430(and channel bottom) will extend from a thread top 440 to a thread bottom 438. The channel 430 may extend all the way around the periphery of the fixture, thus forming an annulus, or may alternatively, extend along part of the periphery. The number of thread bottoms 438 and tops 440 passed by the channel 430 will depend on the lead of the thread 450, the extent of the channel 430 and the angle of the plane P relative to the longitudinal axis X (since the plane P may be inclined at an angle which is different from what is illustrated in the drawing). Apart from the illustrated channel 430, the fixture may be provided with a plurality of other such channels lying in planes being parallel or non-parallel with the indicated plane P. The plurality of channels may describe a full annular path or, alternatively, extend partly around the fixture body. The features and alternatives discussed in connection with the embodiment of Fig. 4 may be combined with above-discussed features and alternatives presented in connection with Figs. 1 to 3. For instance, a fixture may comprise annular channels 430 as well as a diamond-like channel pattern.

Fig. 5a illustrates a fixture 510 according to at least a third example embodiment of the invention. In the enlarged cut-out portion of Fig. 5a, it can be seen that a plurality of channels 530a, 530b are provided in a grid-like tight pattern, such that only pyramidal peaks 560 are formed on the flank surface. This provides for a large area of continuous blood canals/gaps between the bone and the fixture. An alternative is illustrated in the enlarged cut-out view shown in Fig. 5b, wherein a plurality of channels 530c are also provided in a grid-like pattern but have varying width, thereby forming bulges 562 on the flank surface. The features discussed in connection with the embodiments of Figs. 5a and 5b may be combined with the above-discussed features and alternatives presented in the connection with Figs. 1 to 4. For instance, a fixture may comprise annular channels as well as the tight grid-like pattern.

Fig. 6 illustrates a fixture 610 according to at least a fourth example embodiment of the invention. The illustrated fixture 610 is provided with microthreads 622 at the coronal portion 612 of the fixture 610 and provided with macrothreads 624 at the apical 616 and intermediate portions 614 of the fixture 610, and a plurality of channels 630 are provided crossing each other, similarly to the illustration in Fig. 2, however, here illustrated with a tighter channel pattern. Fig. 6 illustrates a socket 660 having an open end in the coronal end 618 of the fixture 610. The socket 660 extends apically into the fixture 610. The socket 660 is for receiving an abutment structure (not shown) which will bridge the gingiva overlying the bore-hole and support/present a prosthetic part. The socket 660 has a conical coronal section 662, an internally threaded apical section (not shown) and an cylindrical or, as illustrated, hexagonal (or other polygonal) intermediate section 664. The conical coronal section 664 may have an angle of taper relative to the longitudinal axis of the fixture of in the range of 7°-15°, such as 10°-12°. The abutment structure will have an apical section which is able to be screw-retained in the fixture socket 660 for releasably securing the abutment structure to the fixture 610, and a conical section for providing a conical seal with coronal section 662 of the socket 660. The features and alternatives discussed in connection with the embodiment of Fig. 6 may be combined with above-discussed features and alternatives presented in the connection with Figs. 1 to 5.

Fig. 7 illustrates a fixture 710 according to at least a fifth example embodiment of the invention. The fixture 710 is provided with wide channels 730. Their width corresponding to about a sixth of the circumference of the fixture 710. The channels 730 are somewhat curved in the apical-to-coronal direction and form a roller-coaster path up and down the thread tops and thread bottoms. The channels 730 may, as conceivable alternatives, be provided with more or less curvature than illustrated, or even be substantially linear in the apical-to-coronal direction. Although the figure illustrates the wide channels 730 as being provided on macrothreads 724 extending along most of the fixture length, such wide channels may be provided on other previously discussed projecting surface structures, such as microthreads, ridges, beads, etc. The features and alternatives discussed in connection with the embodiment of Fig. 7 may be combined with above-discussed features and alternatives presented in the connection with Figs. 1 to 6.

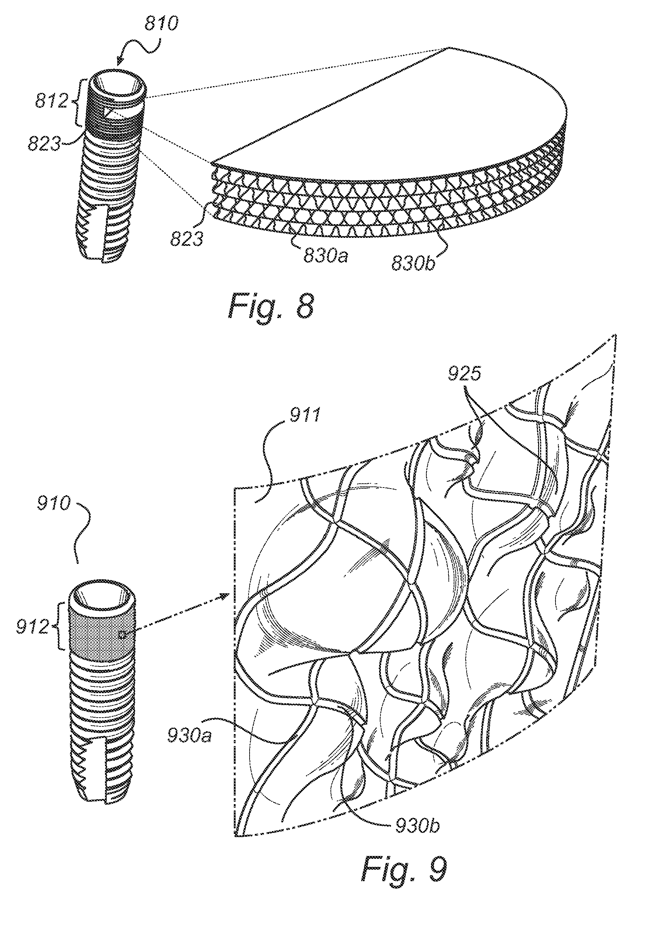

Fig. 8 illustrates a fixture 810 according to an example embodiment which does not fall within the scope of the claims. At a coronal portion 812, the fixture 810 is provided with circumferentially extending ridges 823 which are radially projecting from the core of the fixture 810. The ridges suitably projects about 0.2 mm or less from the core, i.e. similarly to microthreads, however, other ridge heights are readily conceivable. Although, in the illustrated example, the ridges 823 are intended to be annular, an alternative would be to have one or more ridges forming part or parts of a circle. As illustrated in the enlarged cut-out view of Fig. 8, channels 830a, 830b are provided in a criss-cross manner over the ridges 823. Furthermore, in the illustrated example, each ridge 823 lies in a plane which is perpendicular to the longitudinal axis of the fixture 810, i.e. the longitudinal axis X is normal to said plane. Alternatively, one or more ridges could extend in a plane which is at another angle to the longitudinal axis or even at a zero angle to the longitudinal axis of the fixture 810. When the fixture 810 is installed in the bone tissue, the dense cortical bone will generally be in contact with the coronal portion 812 of the fixture 810. Since the blood supply is relatively low in the cortical bone, it may be advantageous to provide the channels 830 at the coronal portion 812 as illustrated in the figure, as this will promote blood distribution. The features and alternatives discussed in connection with the embodiment of Fig. 8 may be combined with above-discussed features and alternatives presented in the connection with Figs. 1 to 7.

Fig. 9 illustrates a fixture 910 according to an example embodiment which does not fall within the scope of the claims. At a coronal portion 912 of the fixture 910 a non-oriented surface structure projecting from the core 911 of the fixture 910 is provided. The surface structure is herein represented by randomly provided bulges 925, as illustrated in the enlarged cut-out view of Fig. 9. Channels 930a, 930b run crosswise in a roller-coaster fashion up and down the bulges 925. The height of a bulge 925 is suitably 0.2 mm or less, however other heights are conceivable. The features and alternatives discussed in connection with the embodiment of Fig. 9 may be combined with above-discussed features and alternatives presented in the connection with Figs. 1 to 8.

## Claims

1. A fixture (10, 210, 310, 510, 610, 710, 810, 910) for insertion into a human bone, comprising
an apical end (20) and a coronal end (18), and
a surface structure projecting from a core (11) of the fixture (10) and having alternating tops (40, 440) and bottoms (38, 438), said surface structure comprising an external thread (22, 24) having a thread profile, and
at least one channel (30, 230a-b, 330a-b, 430, 530a-c, 630, 730, 830a-b, 930a-b) provided in the surface of the thread, the channel having a channel bottom (32), wherein the channel bottom extends along a path extending from the core of the fixture towards a thread top (40), wherein the channel continues over and past the thread top and then extends down towards the core again,
wherein the depth of the channel is smaller than the height of the thread, wherein the profile of the thread comprises coronally facing flanks (34, 234) and apically facing flanks (36, 236), and wherein the channel (30, 230a-b) extends on at least an apically facing thread flank,
wherein the fixture has a longitudinal axis, and
wherein the channel bottom (32) substantially follows the variation of the thread profile relative to said core of the fixture in such a way that when a radial distance from the longitudinal axis of the fixture to the thread profile increases, the radial distance from said longitudinal axis to the channel bottom also increases, **characterised in that** the depth of said at least one channel (30, 230a-b, 330a-b, 430, 530a-c, 630, 730, 830a-b, 930a-b) is 0.05-0.15 mm.

2. The fixture as claimed in claim 1, wherein said at least one channel is in the form of a plurality of channels (30), wherein at least two or more of said plurality of channels extend in parallel with each other.

3. The fixture as claimed in any one of claims 1-2, wherein said at least one channel is in the form of a plurality of channels (230a-b), wherein at least two or more of said plurality of channels extend non-parallelly relative to each other.

4. The fixture as claimed in claim 3, wherein at least one of said channels (230a) crosses at least another one of said channels (230b).

5. The fixture as claimed in any one of claims 1-4, wherein said at least one channel comprises a plurality of channels (530a-b) that form a square-like or diamond-like pattern.

6. The fixture as claimed in any one of claims 1-5, where said at least one channel (430) extends substantially in the circumferential direction of the fixture.

7. The fixture as claimed in claim 6, wherein at least one channel (430) forms an annular path around the core of the fixture.

8. The fixture as claimed in any one of claims 1-7, wherein the distance between the core of the fixture and the channel bottom alternatingly and repeatedly increases and decreases along the extension of the channel (30, 530a-c).

9. The fixture as claimed in any one of claims 1-8, wherein the channel represents a linear, a circular or a helical shape which has been projected onto the surface of the fixture.

10. The fixture as claimed in any one of claims 1-9, wherein the depth of said at least one channel is 0.05 mm - 0.1 mm.

11. The fixture as claimed in any one of claims 1-10, wherein the depth of said at least one channel is smaller than the height of the surface structure, suitably the depth of said at least one channel is 50% or less than 50% of the height of the surface structure, for instance 30% or less than 30% of the height of the surface structure, such as 15%-30% of the height of the surface structure.

12. The fixture as claimed in any one of claims 1-11, wherein the fixture comprises an anchoring portion adapted to be fully inserted into the bone, wherein more than 1 % of the total circumferential surface area of the anchoring portion is provided with/constituted by said at least one channel, suitably more than 5%, for instance more than 10%, such as more than 20%.

13. The fixture as claimed in any one of claims 1-12, wherein said surface structure projecting from the core of the fixture is in the form of a circumferentially-oriented roughness, such as one or more helical threads, circumferential line(s) of beads, circumferentially-oriented ridge(s), or a combination thereof.

14. The fixture as claimed in any one of claims 1-13, wherein said thread extends in the form of a spiral around the longitudinal axis of the fixture, wherein the extension of the channel is nonparallel with said spiral.

15. The fixture as claimed in any one of claims 1-14, wherein the fixture is a dental fixture for insertion into a human jawbone.

## Patentansprüche

1. Befestigungsvorrichtung (10, 210, 310, 510, 610, 710, 810, 910) zum Einsetzen in einen menschlichen Knochen, umfassend:
ein apikales Ende (20) und ein koronales Ende (18), und
eine Oberflächenstruktur, die von einem Kern (11) der Befestigungsvorrichtung (10) vorsteht und abwechselnde Höhen (40, 440) und Tiefen (38, 438) aufweist, wobei die Oberflächenstruktur ein Außengewinde (22, 24) umfasst, das ein Gewindeprofil aufweist, und
mindestens einen Kanal (30, 230a-b, 330a-b, 430, 530a-c, 630, 730, 830a-b, 930a-b), der in der Oberfläche des Gewindes bereitgestellt wird, wobei der Kanal einen Kanalboden (32) aufweist, wobei sich der Kanalboden auf einem Weg erstreckt, der sich von dem Kern der Befestigungsvorrichtung in Richtung auf eine Gewindehöhe (40) erstreckt, wobei der Kanal über und an der Gewindehöhe vorbei fortfährt und sich dann wieder nach unten in Richtung auf den Kern erstreckt,
wobei die Tiefe des Kanals geringer als die Höhe des Gewindes ist, wobei das Profil des Gewindes koronal zugewandte Flanken (34, 234) und apikal zugewandte Flanken (36, 236) umfasst, und wobei sich der Kanal (30, 230a-b) auf mindestens einer apikal zugewandten Gewindeflanke erstreckt,
wobei die Befestigungsvorrichtung eine Längsachse aufweist, und
wobei der Kanalboden (32) im Wesentlichen der Variation des Gewindeprofils im Verhältnis zu dem Kern der Befestigungsvorrichtung derart folgt, dass wenn ein radialer Abstand von der Längsachse der Befestigungsvorrichtung zum Gewindeprofil zunimmt, der radiale Abstand von der Längsachse zum Kanalboden ebenfalls zunimmt,
**dadurch gekennzeichnet, dass**
die Tiefe des mindestens einen Kanals (30, 230a-b, 330a-b, 430, 530a-c, 630, 730, 830a-b, 930a-b) 0,05 bis 0,15 mm beträgt.

2. Befestigungsvorrichtung nach Anspruch 1, wobei der mindestens eine Kanal in Form einer Vielzahl von Kanälen (30) vorliegt, wobei sich mindestens zwei oder mehrere der Vielzahl von Kanälen parallel zueinander erstrecken.

3. Befestigungsvorrichtung nach einem der Ansprüche 1 bis 2, wobei der mindestens eine Kanal in Form einer Vielzahl von Kanälen (230a-b) vorliegt, wobei sich mindestens zwei oder mehrere der Vielzahl von Kanälen im Verhältnis zueinander nicht parallel erstrecken.

4. Befestigungsvorrichtung nach Anspruch 3, wobei mindestens einer der Kanäle (230a) mindestens einen anderen der Kanäle (230b) kreuzt.

5. Befestigungsvorrichtung nach einem der Ansprüche 1 bis 4, wobei der mindestens eine Kanal eine Vielzahl von Kanälen (530a-b) umfasst, die ein quadratartiges oder rautenartiges Muster bilden.

6. Befestigungsvorrichtung nach einem der Ansprüche 1 bis 5, wobei sich der mindestens eine Kanal (430) im Wesentlichen in der Umfangsrichtung der Befestigungsvorrichtung erstreckt.

7. Befestigungsvorrichtung nach Anspruch 6, wobei mindestens ein Kanal (430) einen ringförmigen Weg um den Kern der Befestigungsvorrichtung herum bildet.

8. Befestigungsvorrichtung nach einem der Ansprüche 1 bis 7, wobei der Abstand zwischen dem Kern der Befestigungsvorrichtung und dem Kanalboden abwechselnd und wiederholt entlang der Erstreckung des Kanals (30, 530a-c) zunimmt und abnimmt.

9. Befestigungsvorrichtung nach einem der Ansprüche 1 bis 8, wobei der Kanal eine lineare, eine kreisförmige oder eine schraubenförmige Form darstellt, die auf die Oberfläche der Befestigungsvorrichtung projiziert wurde.

10. Befestigungsvorrichtung nach einem der Ansprüche 1 bis 9, wobei die Tiefe des mindestens einen Kanals 0,05 mm bis 0,1 mm beträgt.

11. Befestigungsvorrichtung nach einem der Ansprüche 1 bis 10, wobei die Tiefe des mindestens einen Kanals kleiner als die Höhe der Oberflächenstruktur ist, die Tiefe des mindestens einen Kanals geeignet 50 % oder weniger als 50 % der Höhe der Oberflächenstruktur, zum Beispiel 30 % oder weniger als 30 % der Höhe der Oberflächenstruktur, wie etwa 15 % bis 30 % der Höhe der Oberflächenstruktur beträgt.

12. Befestigungsvorrichtung nach einem der Ansprüche 1 bis 11, wobei die Befestigungsvorrichtung einen Verankerungsabschnitt umfasst, der geeignet ist, um vollständig in den Knochen eingesetzt zu werden, wobei mehr als 1 % der gesamten Umfangsmantelfläche des Verankerungsabschnitts mit dem mindestens einen Kanal versehen ist bzw. daraus besteht, bevorzugt mehr als 5 %, beispielsweise mehr als 10 %, wie etwa mehr als 20 %.

13. Befestigungsvorrichtung nach einem der Ansprüche 1 bis 12, wobei die Oberflächenstruktur, die von dem Kern der Befestigungsvorrichtung vorsteht in Form einer umfangsmäßig orientierten Unebenheit vorliegt, wie etwa als ein oder mehrere Schraubengewinde, eine oder mehrere Umfangslinien von Wülsten, eine oder mehrere umfangsmäßig orientierte Rippen oder eine Kombination davon.

14. Befestigungsvorrichtung nach einem der Ansprüche 1 bis 13, wobei sich das Gewinde in Form einer Spirale um die Längsachse der Befestigungsvorrichtung herum erstreckt, wobei die Erstreckung des Kanals zu der Spirale nicht parallel ist.

15. Befestigungsvorrichtung nach einem der Ansprüche 1 bis 14, wobei die Befestigungsvorrichtung ein Zahnimplantat zum Einsetzen in einen menschlichen Kieferknochen ist.

## Revendications

1. Implant (10, 210, 310, 510, 610, 710, 810, 910) destiné à être inséré dans un os humain, comprenant :
une extrémité apicale (20) et une extrémité coronale (18) et
une structure surfacique saillant depuis un noyau (11) de l'implant (10) et comportant des hauts (40, 440) et des bas (38, 438) alternants, ladite structure surfacique comportant un filet externe (22, 24) doté d'un profil de filet, et
au moins un canal (30, 230a-b, 330a-b, 430, 530a-c, 630, 730, 830a-b, 930a-b) prévu dans la surface du filet, le canal comportant un fond de canal (32), le fond de canal s'étendant le long d'un passage s'étendant depuis le noyau de l'implant vers un haut de filet (40), le canal se continuant à travers et au-delà du haut de filet puis s'étendant à nouveau vers le bas vers le noyau,
dans lequel la profondeur du canal est inférieure à la hauteur du filet, dans lequel le profil du filet comprend des flancs opposés au niveau coronal (34, 234) et des flancs opposés au niveau (36, 236) et dans lequel le canal (30, 230a-b) s'étend sur au moins un flanc opposé au niveau apical,
dans lequel l'implant a un axe longitudinal et
dans lequel le fond de canal (32) suit sensiblement la variation du profil du filet par rapport audit noyau de l'implant de manière à ce que, lorsqu'une distance radiale entre l'axe longitudinal de l'implant et le profil du filet augmente, la distance radiale entre ledit axe longitudinal et le fond du canal augmente également,
**caractérisé en ce que**
la profondeur dudit au moins un canal (30, 230a-b, 330a-b, 430, 530a-c, 630, 730, 830a-b, 930a-b) est de 0,05 à 0,15 mm.

2. Implant selon la revendication 1, dans lequel ledit au moins un canal prend la forme d'une pluralité de canaux (30) et dans lequel au moins deux ou plus parmi ladite pluralité de canaux s'étendent parallèlement les uns aux autres.

3. Implant selon l'une quelconque des revendications 1 à 2, dans lequel ledit au moins un canal prend la forme d'une pluralité de canaux (230a-b) et dans lequel au moins deux ou plus parmi ladite pluralité de canaux s'étendent non parallèlement les uns aux autres.

4. Implant selon la revendication 3, dans lequel au moins un desdits canaux (230a) croise au moins un autre desdits canaux (230b).

5. Implant selon l'une quelconque des revendications 1 à 4, dans lequel ledit au moins un canal comprend une pluralité de canaux (530a-b) qui forment un motif ressemblant à un carré ou à un diamant.

6. Implant selon l'une quelconque des revendications 1 à 5, dans lequel ledit au moins un canal (430) s'étend sensiblement dans le sens circonférentiel de l'implant.

7. Implant selon la revendication 6, dans lequel ledit au moins un canal (430) forme un passage annulaire autour du noyau de l'implant.

8. Implant selon l'une quelconque des revendications 1 à 7, dans lequel la distance entre le noyau de l'implant et le fond du canal augmente et diminue alternativement à répétition le long de l'extension du canal (30, 530a-c).

9. Implant selon l'une quelconque des revendications 1 à 8, dans lequel le canal représente une forme linéaire, circulaire ou hélicoïdale qui a été projetée sur la surface de l'implant.

10. Implant selon l'une quelconque des revendications 1 à 9, dans lequel la profondeur dudit au moins un canal est de 0,05 mm à 0,1 mm.

11. Implant selon l'une quelconque des revendications 1 à 10, dans lequel la profondeur dudit au moins un canal est inférieure à la hauteur de la structure surfacique, de manière adéquate la profondeur dudit au moins un canal représente 50 % ou moins de 50 % de la hauteur de la structure surfacique, par exemple 30 % ou moins de 30 % de la hauteur de la structure surfacique, comme 15 % à 30 % de la structure surfacique.

12. Implant selon l'une quelconque des revendications 1 à 11, dans lequel l'implant comprend une section d'ancrage apte à être entièrement insérée dans l'os, dans lequel plus de 1 % de l'aire de surface circonférentielle totale de la section d'ancrage est équipée/constituée dudit au moins un canal, de manière adéquate plus de 5 %, par exemple plus de 10 %, comme plus de 20 %.

13. Implant selon l'une quelconque des revendications 1 à 12, dans lequel ladite structure surfacique saillant depuis le noyau de l'implant prend pour forme une rugosité orientée vers la circonférence, comme un ou plusieurs filets hélicoïdaux, une ou plusieurs lignes de bourrelets circonférentiels, une ou plusieurs côtes à orientation circonférentielle ou leurs combinaisons.

14. Implant selon l'une quelconque des revendications 1 à 13, dans lequel ledit filet s'étend en forme de spirale autour de l'axe longitudinal de l'implant, dans lequel l'extension du canal est non parallèle à ladite spirale.

15. Implant selon l'une quelconque des revendications 1 à 14, dans lequel l'implant est un implant dentaire destiné à être inséré dans un maxillaire humain.
